# EUROPÄISCHE PATENTSCHRIFT

(11) **EP 1 624 960 B1**
(45) Veröffentlichungstag und Bekanntmachung des Hinweises auf die Patenterteilung: **06.08.2008**
(21) Anmeldenummer: 04715883.7
(22) Anmeldetag: 01.03.2004
(51) Int. Cl.: B01J 19/12

(54) **VORRICHTUNG ZUM EINF HREN VON ELEKTROMAGNETISCHER STRAHLUNG IN EINEN REAKTOR**
DEVICE FOR INJECTING ELECTROMAGNETIC RADIATION INTO A REACTOR
DISPOSITIF POUR INJECTER UN RAYONNEMENT ELECTROMAGNETIQUE DANS UN REACTEUR

(30) Priorität: 30.04.2003 DE 10319811
(43) Veröffentlichungstag der Anmeldung: 15.02.2006
(73) Patentinhaber: Uhde GmbH, 44141 Dortmund (DE)
(72) Erfinder: BENJE, Michael, 64289 Darmstadt (DE)
(74) Vertreter: Ackermann, Joachim
(86) Internationale Anmeldenummer: PCT/EP2004/002023
(87) Internationale Veröffentlichungsnummer: WO 2004/096431

(56) Entgegenhaltungen:
- WO-A-03/093207
- DE-A- 3 008 848
- US-A- 3 969 204
- US-A- 4 595 579

## Beschreibung

Die vorliegende Erfindung betrifft eine Vorrichtung mit der elektromagnetische Strahlung in einen Reaktor eingekoppelt werden kann, insbesondere zur photolytischen Radikalerzeugung sowie einen damit ausgestatteten Reaktor zum Durchführen radikalischer Kettenreaktionen in der Gasphase.

Bei der Durchführung radikalischer Kettenreaktionen ist es häufig erwünscht, große Mengen an Starterradikalen zur Verfügung zu haben, um den Umsatz in einem gegebenen Reaktionsvolumen unter ansonsten gleichen Bedingungen zu erhöhen.

Radikalische Gasphasenreaktionen werden seit vielen Jahren großtechnisch betrieben. Eine der Maßnahmen zur Verbesserung der Wirtschaftlichkeit der Verfahren könnte darin bestehen, möglichst hohe Reaktionsumsätze anzustreben. Zu diesem Zweck hat man dem Eduktgas bereits sogenannte Promotoren zugesetzt. Dabei handelt es sich um Verbindungen, die unter den im Reaktor herrschenden Bedingungen in Radikale zerfallen und in die Kettenreaktion, die zur Bildung der gewünschten Produkte führt, eingreifen. Der Einsatz derartiger Verbindungen ist beispielsweise aus der US-A-4,590,318 oder der DE-A-3,328,691 bekannt.

Es ist bereits vorgeschlagen worden, halogenhaltige aliphatische Kohlenwasserstoffe mit Hilfe von Laserlicht in Radikale zu spalten und diese in Radikalkettenreaktionen, wie zur Darstellung von Vinylchlorid, einzusetzen. Beispiele dafür finden sich in SPIE, Vol. 458 Applications of Lasers to Industrial Chemistry (1984), S. 82-88, in Umschau 1984, Heft 16, S. 482 sowie in den DE-A-2,938,353, DE-C-3,008,848 und EP-A-27,554. Bis heute hat diese Technik allerdings keinen Eingang in die industrielle Produktion gefunden. Ein Grund mag darin liegen, dass sich die bislang vorgeschlagenen Reaktoren für einen Dauerbetrieb nicht eignen.

Bei der Durchführung radikalischer Gasphasenreaktionen hat man mit der Bildung von Nebenprodukten zu rechnen. Diese verunreinigen den Reaktor während des Dauerbetriebs und schlagen sich im Reaktor beispielsweise als Koks nieder.

Besonders beim Einsatz von Promotorsubstanzen erhöht sich die Koksbildungsrate, da diese in Mengen eingesetzt werden müssen, durch die bereits ein merklicher Eingriff in das Reaktionssystem erfolgt.

Diese Nachteile kompensieren den durch die Umsatzerhöhung gewonnenen wirtschaftlichen Vorteil und führen dazu, dass die Anwendung von Promotorsubstanzen sich bis heute in der industriellen Praxis nicht durchsetzen konnte.

Die Problematik der Koksbildung wurde bereits in der DE-A-30 08 848 beschrieben. Dort wird die photochemische Initiierung einer Radikalkettenreaktion durch direkte Einstrahlung von Licht in den Reaktionsraum vorgeschlagen, und zwar sowohl mit Metalldampflampen als auch mit Lasern als Lichtquelle. Dort wird auch die Beobachtung beschrieben, dass sich bei Verwendung kontinuierlich arbeitender Lichtquellen, wie Metalldampflampen, das Fenster rasch mit Nebenprodukten bedeckt, während es bei Verwendung von Lasern frei bleibt.

Als Abhilfemaßnahme wird das Arbeiten mit hoher Strömungsgeschwindigkeit im Bereich des optischen Fensters vorgeschlagen, damit die gebildeten Nebenprodukte erst stromabwärts vom Fenster in merklicher Menge gebildet werden.

An dieser Vorgehensweise ist jedoch von Nachteil, dass die "Selbstreinigung" des Fensters wahrscheinlich auf die Verwendung von gepulsten Lasern beschränkt ist, da hier durch eine kurzzeitige lokale Erhitzung des Gases in und um die Kokspartikel Druckstöße erzeugt werden, welche die Kokspartikel bzw. die Koksschicht dann von Fenster absprengen. Die Verwendung von gepulsten Lasern wird zwar in der DE-A-30 08 848 nicht explizit erwähnt, wohl aber in der DE-A-29 38 353, auf die in der DE-A-30 08 848 ausdrücklich Bezug genommen wird.

Die den DE-A-30 08 848 und DE-A-29 38 353 zugrunde liegenden Experimente wurden in Quarzreaktoren durchgeführt. In industriellen Reaktoren aus Metall wird jedoch schon im Eintrittsbereich des Reaktors und damit "stromaufwärts" eines möglicherweise angebrachten optischen Fensters Koks gebildet. Mögliche Ursachen hierfür sind einerseits, dass schon im Eintrittsbereich das Reaktors Koksprekursoren gebildet werden und andererseits darin, dass auch bei sorgfältiger destillativer Reinigung der Edukte im industriellen Verfahren mit den Edukten geringe Mengen an Koksprecursoren in den Reaktor eingetragen werden. Es besteht daher ein Bedarf nach weiteren Verfahren, die sich in einfacher Weise in die industrielle Praxis umsetzen lassen und bei denen eine Koksbildung wirksam vermieden werden kann.

Mit der vorliegenden Erfindung werden diese Nachteile umgangen und es werden eine Vorrichtung bzw. ein Reaktor zur Verfügung gestellt, bei denen Licht in einen unter den Bedingungen der thermischen Spaltung von Kohlenwasserstoffen betriebenen Reaktor eingekoppelt werden kann. Dazu wird einem vom eigentlichen Reaktionsraum abgetrennten, von der Strahlungsquelle durchstrahlten Kompartment ein Spülgas zugeführt, das sodann in den Reaktionsraum eingebracht wird.

Eine Aufgabe der vorliegenden Erfindung ist das Bereitstellen einer Vorrichtung zur Einkopplung von elektromagnetischer Strahlung in einen Reaktor, die im Dauerbetrieb eingesetzt werden kann, und bei der die Neigung zur Koksbildung bzw. zum Absetzen von Koks auf den optischen Fenstern oder Lichtleitern im Vergleich zu bekannten Vorrichtungen deutlich herabgesetzt ist.

Eine weitere Aufgabe der vorliegenden Erfindung besteht in der Bereitstellung eines Reaktors, der auch unter Bedingungen hoher Temperaturen auf einfache Weise gasdicht mit einer Vorrichtung zur Einkopplung von elektromagnetischer Strahlung verbunden werden kann.

Noch eine weitere Aufgabe der vorliegenden Erfindung besteht in der Bereitstellung einer Vorrichtung sowie eines Reaktors zur Durchführung von radikalischen Kettenreaktionen, bei denen im Vergleich zu herkömmlichen Reaktoren bei ansonsten gleicher Betriebstemperatur größere Umsätze erzeugt werden können oder bei denen im Vergleich zu herkömmlichen Reaktoren bei ansonsten gleichen Umsätze eine Absenkung der Betriebstemperatur, möglich ist.

Die vorliegende Erfindung betrifft eine Vorrichtung zum Einkoppeln von elektromagnetischer Strahlung in einen Reaktor zur Durchführung radikalischer Gasphasenreaktionen, umfassend die Elemente:
a) ein vom Reaktionsraum des Reaktors abgetrenntes Kompartment, das
b) mit dem Reaktionsraum des Reaktors über mindestens eine Öffnung in Verbindung steht,
c) mindestens eine Zuleitung zum Einleiten eines Spülgases in das Kompartment, das ein Inertgas und/oder ein Spaltpromotoren enthaltendes Gas und/oder ein gasförmiges Reduktionsmittel ist, so dass sich ein Gaspolster ausbildet, welches die optischen Bauteile chemisch weitgehend vom Reaktionsraum abkoppelt, sowie
d) mindestens eine Quelle von elektromagnetischer Strahlung, die so angeordnet ist, dass
e) die elektromagnetische Strahlung das Kompartment und den daran sich anschließenden Reaktionsraum des Reaktors durchstrahlt.

Die erfindungsgemäße Vorrichtung ist mit einer in das Kompartment mündenden Rohrleitung für das Spülgas verbunden.
Durch die erfindungsgemäße Vorrichtung wird ein Spülgas in den Reaktor eingeleitet.

Dabei kann es sich um ein Inertgas handeln und/oder um ein Spaltpromotoren enthaltendes Gas und/oder um gasförmige Reduktionsmittel.

Im Falle von Spaltpromotoren erfolgt deren photolytische Spaltung im Kompartment unmittelbar vor der Einleitung in den Reaktor, bzw. im durchstrahlten Bereich hinter der Mündung des Kompartments in den Reaktor.

Beispiele für Inertgase sind unter den im Reaktor herrschenden Reaktionsbedingungen inerte Gase, wie Stickstoff, Edelgase, z.B. Argon, oder Kohlendioxid.

Ein Beispiel für ein gasförmiges Reduktionsmittel ist Wasserstoff.

Promotoren zum Einsatz für die photolytische Zersetzung in der erfindungsgemäßen Vorrichtung und zur Einleitung in den Reaktor sind an sich bekannt. Dabei handelt es sich um Verbindungen, die unter Strahlungseinfluss in aktive Spezies, wie Radikale, zerfallen und den Ablauf der radikalischen Kettenreaktion im Reaktionsraum des Reaktors fördern.

Typische Temperaturen des Gasstromes im Innern des Reaktors am Ort der Vorrichtung zur Einkopplung von elektromagnetischer Strahlung sind von der Art der Gasphasenreaktion abhängig und umfassen einen weiten Bereich, beispielsweise von 250 bis 1300°C.

Der durch das über die erfindungsgemäße Vorrichtung zugeleitete Gas hervorgerufene Effekt ist neben der gewählten Temperatur auch von der Natur der erzeugten Radikale und auch von deren Menge abhängig. Üblicherweise setzt man insgesamt nicht mehr als 10 Gew. % an Spülgas, vorzugsweise nicht mehr als 5 Gew. %, insbesondere 0,0005 bis 5 Gew. %, bezogen auf den Gesamtmassenstrom im Reaktor zu.

Es wird angenommen, dass die Zuführung von Spülgas die Ablagerung von Koks auf den Teilen verhindert, die sich im optischen Weg befinden.

Beim Einsatz von Promotoren werden darüber hinaus noch Radikale photolytisch erzeugt, was den Verlauf der Radikalkettenreaktion im Reaktionsgemisch fördert, da letztendlich eine erhöhte Konzentration von Radikalen zur Verfügung steht.

Bevorzugt befindet sich mindestens eine erfindungsgemäße Vorrichtung in der Nähe des Eintritts des Eduktgasstromes in den Reaktor. Dadurch wird bereits bei Eintritt des Eduktgases in den Reaktor eine hohe Konzentration an Radikalen gebildet, die zu einem effizienten Verlauf der Kettenreaktion beitragen.

In einer bevorzugten Variante des erfindungsgemäßen Reaktors kommt das Reaktionsgemisch beim Durchlauf im Reaktor mit mehreren erfindungsgemäßen Vorrichtungen in Kontakt.

Ganz besonders bevorzugt ist die Anzahl der erfindungsgemäßen Vorrichtungen im ersten Drittel des Reaktors größer als im zweiten Drittel und/oder im dritten Drittel.

Die Erfindung betrifft auch einen Reaktor zur Durchführung von radikalischen Gasphasenreaktionen, in den mindestens eine der oben definierten Vorrichtungen mündet.

Der Reaktor umfasst mindestens folgende Elemente:
i) in den Reaktor mündende Zuleitung für den Eduktgasstrom,
ii) mindestens eine oben definierte und ein Kompartment umfassende und in den Reaktor mündende Vorrichtung zur Einkopplung von elektromagnetischer Strahlung in den Reaktor
iii) in das Kompartment der Vorrichtung mündende Zuleitung für ein Spülgas, das ein Inertgas und/oder ein Spaltpromotoren enthaltendes Gas und/oder ein gasförmiges Reduktionsmittel ist, so dass sich ein Gaspolster ausbildet, welches die optischen Bauteile chemisch weitgehend vom Reaktionsraum abkoppelt, so
iv) mit der Zuleitung verbundene Quelle für ein Spülgas,
v) gegebenenfalls Heizvorrichtung für das Aufheizen des Spülgases in der Zuleitung,
vi) Heizvorrichtung für das Aufheizen und/oder die Aufrechterhaltung der Temperatur des Gasstromes im Reaktor, und
vii) aus dem Reaktor führende Ableitung für den Produktgasstrom der radikalischen Gasphasenreaktion.

Als Reaktor können alle dem Fachmann für derartige Reaktionen bekannten Typen eingesetzt werden. Bevorzugt wird ein Rohrreaktor.

Dem erfindungsgemäßen Reaktor kann ein adiabatischer Nachreaktor nachgeschaltet sein, der vorzugsweise die oben definierten Elemente ii), iii) und iv) enthält. In dem adiabatischen Nachreaktor wird die benötigte Reaktionswärme durch die Wärme des zugeführten Produktgasstromes geliefert, der sich dadurch abkühlt.

Anstelle der Kombination des erfindungsgemäßen Reaktors mit einem adiabatischen Nachreaktor enthaltend die Elemente ii), iii) und iv) kann ein solcher adiabatischer Nachreaktor auch mit einem an sich bekannten Reaktor verschaltet sein, der die Elemente ii), iii) und iv) nicht aufweist.

Die erfindungsgemäße Vorrichtung mündet in das Innere des erfindungsgemäßen Reaktors und speist in den Eduktgasstrom bei dessen Durchlauf durch den Reaktor ein Spülgas ein und gestattet die Einkopplung von elektromagnetischer Strahlung in das Innere des Reaktors.
Das dem eigentlichen Reaktionsraum vorgeschaltete Kompartment wird vom Spülgas(gemisch) durchströmt und tritt dann durch eine Öffnung, die als Düse ausgestaltet sein kann, in den eigentlichen Reaktionsraum ein.

Zur Durchführung der Photolyse wird elektromagnetische Strahlung aus einer für die beschriebenen Zwecke geeigneten Strahlungsquelle durch ein optisch durchlässiges Fenster, vorzugsweise ein Quarzfenster, oder einen Lichtleiter in ein von eigentlichen Reaktionsraum abgetrenntes Kompartment eingekoppelt und durchstrahlt das Kompartment selbst und auch einen Teil des angrenzenden Reaktionsraumes.

Im Kompartment bildet das Spülgas ein Gaspolster aus, welches die optischen Bauteile, z.B. das optische Fenster, chemisch weitgehend vom Reaktionsraum abkoppelt. Der Zweck dieser Maßnahme soll im folgenden an einem Beispiel näher erläutert werden.

Eine unerwünschte Nebenreaktion bei der Durchführung radikalischer Gasphasenreaktionen ist die Ablagerung von Koks auf den Reaktorwänden. Der Prozess der Koksabscheidung verläuft auf nichtmetallischen Werkstoffen wie z.B. Quarz langsamer als auf metallischen Werkstoffen. Diese Tatsache macht man sich in neuerer Zeit zunutze, um die Koksbildung in Reaktorrohren durch das Aufbringen nichtmetallischer Überzüge auf die Rohrinnenwand zu verlangsamen. Trotz dieser Tatsache würde sich Koks auch auf den optischen Bauteilen abscheiden, sofern diese direkt dem Reaktionsgemisch ausgesetzt wären, also z.B. direkt in die Reaktorwand eingesetzt wären.

Mit der vorliegenden Erfindung werden die Nachteile vorbekannter Verfahrensweisen umgangen und es wird eine Vorrichtung bzw. ein Reaktor zur Verfügung gestellt, bei denen elektromagnetische Strahlung in einen unter den Bedingungen der radikalischen Gasphasenreaktion betriebenen Reaktor eingekoppelt werden kann.

Besonders bevorzugte Ausführungsformen der erfindungsgemäßen Vorrichtung sowie deren bevorzugte Anordnung in einem Reaktor werden nachstehend anhand der Figuren 1, 2, 3 und 4 beschrieben.

Es zeigen
Figur 1: Eine bevorzugte Vorrichtung zur Erzeugung von Radikalen aus Spaltpromotoren durch elektromagnetische Strahlung und zum Einleiten des Spülases in einen radikalischen Gasphasenreaktor dargestellt im Längsschnitt
Figur 2: Eine Modifikation der Vorrichtung nach Figur 1 dargestellt im Längsschnitt
Figur 3: Eine weitere Modifikation der Vorrichtung nach Figur 1 dargestellt im Längsschnitt
Figur 4: Rohrreaktor mit Vorrichtung gemäß Figur 1 im Längsschnitt

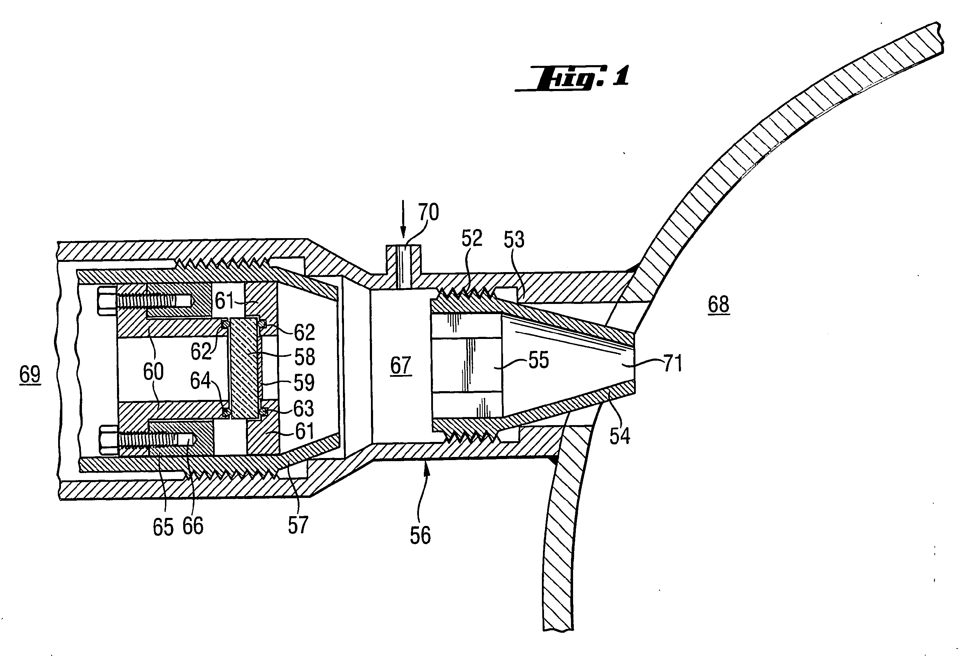

In Figur 1 sind eine bevorzugt eingesetzte Vorrichtung zur Einkopplung von elektromagnetischer Strahlung und deren Anbringung im erfindungsgemäßen Reaktor dargestellt.

An einen Bogen des Reaktionsrohrs ist eine Halterung angeschweißt, die in ihrem Inneren ein Gewinde (52) sowie eine umlaufende Dichtkante (53) aufweist. In diese Halterung kann eine konische Hülse (54) eingeschraubt werden, deren vorderes Ende als Düse ausgebildet sein kann und die zwecks besserer Verschraubbarkeit z.B. einen Innensechskant (55) aufweisen kann. Wird die konische Hülse (54) in die Halterung (56) eingeschraubt, so bildet diese mit der Dichtkante (53) der Halterung eine unter den Bedingungen der Reaktion zuverlässige Abdichtung. Dieses bewährte Dichtprinzip wurde bereits in der DE-A-44 20 368 beschrieben. Ebenfalls wie in DE-A-44 20 368 bereits beschrieben kann eine zusätzliche Abdichtung durch eine (in der Figur 1 nicht dargestellte) Stopfbuchspackung erfolgen.

In die Halterung (56) kann unter Ausnutzung des selben Dichtprinzips eine weitere Hülse (57) eingeschraubt werden, die ein optisch durchlässiges Fenster (58) enthält, z. B. ein Quarzfenster, das mit einer optisch halbdurchlässigen Metallschicht (59) beschichtet sein kann, wobei das Metall vorzugsweise ein Hydrierkatalysator und ganz besonders bevorzugt ein Platinmetall ist.

Das optische Fenster ist zwischen Halterungen (60, 61) eingespannt, die an ihren dem Fenster zugewandten Seiten umlaufende Aussparungen (62) aufweisen, die jeweils eine Dichtung (63, 64), vorzugsweise eine Metalldichtung und ganz besonders bevorzugt eine Golddichtung aufnehmen.

Das Fenster (58) wird von der Halterung (60) gegen die Halterung (61) gepresst. Dies kann durch Verschraubung der Halterung (60) mit einem Lagerring oder Lagerblöcken (65) geschehen, der z.B. mit Sacklöchern (66) versehen ist.

Die Halterungen (60) und (61), die Aussparungen (62) und die dickere der Dichtungen sind so dimensioniert, dass sich beim Verschrauben der Anordnung eine definierte Flächenpressung der Dichtungen einstellt und das optische Fenster nicht beschädigt wird.

Der Zwischenraum (67) zwischen den Hülsen (54) und (57) ist mit einer oder mehreren Gaszuleitungen versehen und bildet ein vom Reaktionsraum (68) sowie vom Außenraum (69) abgetrenntes Kompartment.

Die radikalische Gasphasenreaktion findet im Reaktionsraum (68) statt. Die gesamte Anordnung ist bei Rohrreaktoren vorzugsweise an einem Bogen des Reaktionsrohrs montiert, der aus der eigentlichen Strahlungszone des Ofens herausragt und von dieser thermisch isoliert ist.

Durch den Gaseinlass (70) strömt ein Spülgas, z.B. Stickstoff oder ein Edelgas, oder ein Gemisch eines Inertgases mit einer Promotorsubstanz oder eine gasförmige Promotorsubstanz in das Kompartment (67). Das Gas verlässt das Kompartment und strömt durch die Öffnung (71) in den Reaktionsraum ein.

Durch die permanente Spülung des Kompartments ist das optische Fenster vom Reaktionsraum (68) durch ein Gaspolster getrennt. Daher können Koksprecursoren, wie Acetylen, Benzol oder Chloropren nicht an das Fenster gelangen und dort Koksablagerungen bilden.

In einer bevorzugten Ausführungsform ist das optische Fenster mit einer optisch halbdurchlässigen Metallschicht beschichtet, wobei das Metall ein Hydrierkatalysator ist, z.B. Palladium.

Wird dem Promotorgas nun eine geringe Menge Wasserstoff zugemischt, so werden Koksvorläufer, die trotz Anspülung bis an das optische Fenster gelangen, an dessen Oberfläche reduziert. Dadurch können sich auf der Oberfläche des Fensters keine Koksablagerungen bilden.

Die Strahlung der Strahlungsquelle durchstrahlt das optische Fenster und überträgt Energie auf die Moleküle des Reaktionsgemisches im Reaktionsraum (68) und auf eine gegebenenfalls im Kompartment (67) vorliegende Promotorsubstanz. Dadurch werden Radikale erzeugt (Photolyse), die dann die im eigentlichen Reaktionsraum (68) ablaufende Reaktion fördern.

Normalerweise ist die Erzeugung von Radikalen und deren anschließender Transport in den Reaktionsraum schwierig, da unter den herrschenden Druckverhältnissen (typischerweise 9 - 25 bar) die Radikale rasch rekombinieren.

Bei der erfindungsgemäßen Anordnung werden jedoch das gesamte Kompartment und auch der Reaktionsraum durchstrahlt. Dies hat zur Folge, dass die gewünschten Radikale auch in der Öffnung (71) und in der dieser Öffnung benachbarten Zone des Reaktionsraums gebildet werden und somit sicher in das Reaktionsgeschehen eingreifen können. Daher sind keine hohen Strömungsgeschwindigkeiten des Spülgases erforderlich.

In einer weiteren Ausführungsform kann die Außenwand der Vorrichtung, insbesondere der Teil der Vorrichtung, der in den Reaktionsraum hineinragt, mit einem inerten Material, z. B. einem Metalloxid, Keramik, Bornitrid oder Siliziumnitrid beschichtet sein.

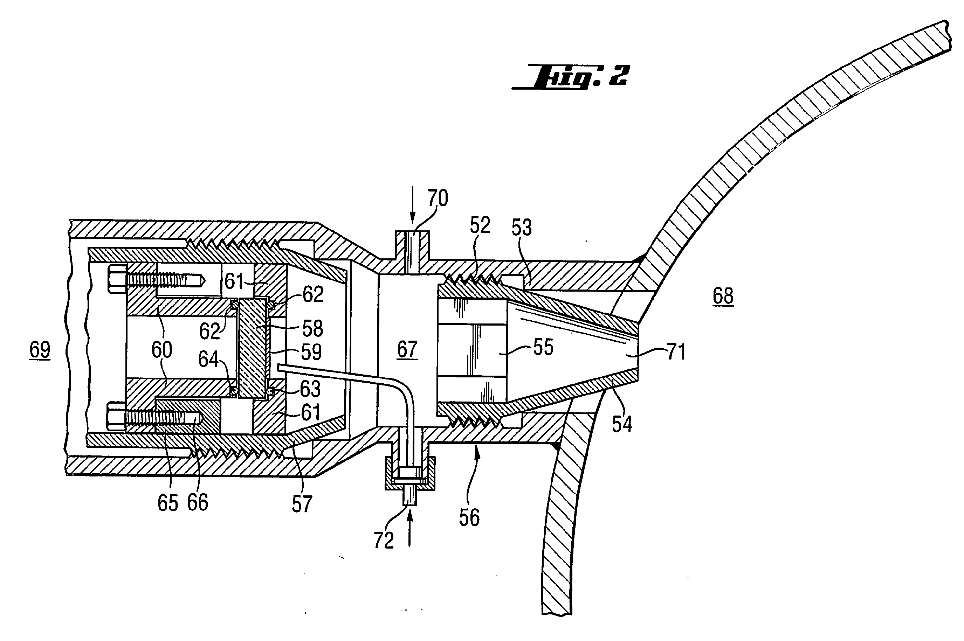

In einer weiteren bevorzugten Ausgestaltung, wie in Figur 2 dargestellt, weist das Kompartment (67) einen weiteren Gaseinlass (72) auf, der bis nahe der Oberfläche des optischen Fensters (58) geführt wird. So ist es möglich, das Fenster und dessen unmittelbare Umgebung mit Gas zu spülen, während ein weiterer Teil an Spülgas durch den Gaseinlass (70) zugegeben werden. Durch eine solche Anordnung lässt sich das optische Fenster noch besser gegen Koksablagerungen schützen.

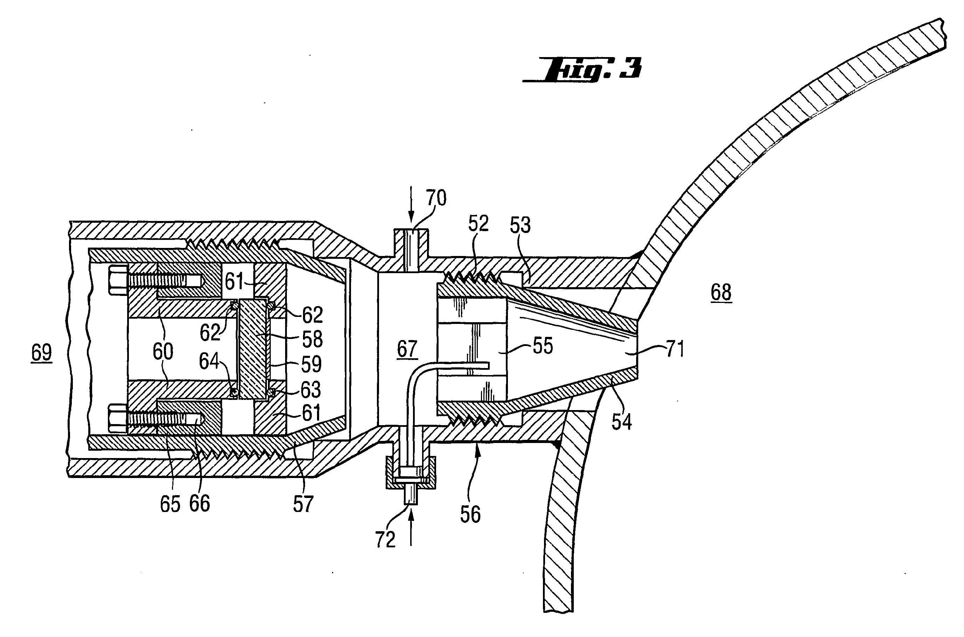

Eine weitere bevorzugte und in Figur 3 dargestellte Ausführungsform ähnelt der in Figur 2 dargestellten Variante. Der weitere Gaseinlass (72) wird hier allerdings in Richtung der Öffnung (71) geführt und dient der Zuführung von gasförmiger Promotorsubstanz. Der Gaseinlass (70) dient lediglich der Zuleitung von Spülgas, das vorzugsweise ein Inertgas ist. Auf diese Weise erfolgt die Erzeugung der Radikale aus der Promotorsubstanz in der Nähe des Reaktionsraumes (68) und entfernt vom optischen Fenster (58). Dadurch ist ein weiterer Schutz des optischen Fensters (58) gegenüber Koksablagerungen möglich.

Als Strahlungsquelle kann jede Vorrichtung verwendet werden, deren Strahlung zur Photolyse von Komponenten des Reaktionsgemisches geeignet ist. Dies kann eine UV-Lampe (z.B. eine Metalldampflampe) oder ein Laser sein. Bei Verwendung von Lasern ist es bei der hier vorgeschlagenen Anordnung ohne Bedeutung, ob ein gepulster Laser oder ein Dauerstrahllaser verwendet wird. Auch Excimer-Lampen können als Lichtquelle verwendet werden.

Die Einkopplung der eingesetzten Strahlung ist auf unterschiedliche Weisen möglich. So kann das Licht z.B. durch ein Lichtleiterbündel eingekoppelt werden. Weiterhin kann die Lichtquelle (z.B. im Falle der Verwendung einer Metalldampf- oder Excimerlampe) direkt in der Hülse (57) hinter dem optischen Fenster eingebaut werden. In diesem Fall ist vorzugsweise eine entsprechende Kühlung vorzusehen. Auch kann das Licht durch ein weiteres Fenster in die Hülse (57) eingekoppelt und durch Spiegel auf das Fenster (58) umgelenkt werden.

In einer besonderen Ausführungsform wird zur Lichteinkopplung eine Vorrichtung analog zu den DE-A-198 45 512 bzw. DE-Gbm-200 03 712 verwendet. Die vorbekannten Vorrichtungen dienen zur Beobachtung von Vorgängen im Brennraum von in Betrieb befindlichen Verbrennungskraftmaschinen und werden z. B. in Form sogenannter Zündkerzenadapter eingesetzt. Neben Ihrem eigentlichen Einsatzzweck, der optischen Beobachtung von Verbrennungsvorgängen, sind solche Vorrichtungen aufgrund Ihrer Druck - und Temperaturfestigkeit ebenso zum Einkoppeln von Licht in chemische Reaktoren geeignet, in denen ähnliche Druck- und Temperaturverhältnisse herrschen wie in laufenden Verbrennungsmotoren.

Falls solche Vorrichtungen eingesetzt wird, kann das in den Figuren 1, 2 und 3 dargestellte optische Fenster mit dem beschriebenen Abdichtungssystem entfallen. Die Lichtzuleitung würde dann in Form eines Adapters analog einem oder mehrerer sog. Zündkerzenadapter in eine in der Hülse (57) befindliche Trennwand eingeschraubt werden.

Die in Figur 1 dargestellte Vorrichtung kann in einen konventionellen Rohrreaktor zur Durchführung radikalischer Gasphasenreaktionen eingebaut werden.

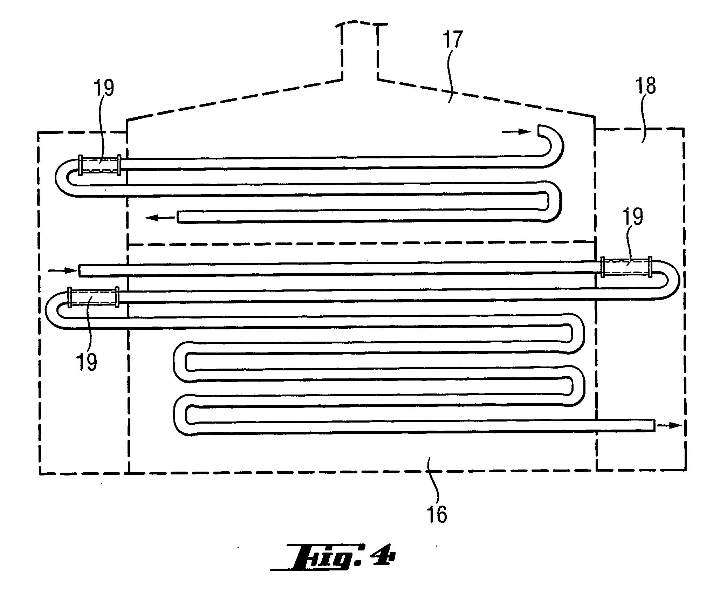

Eine mögliche Anordnung der erfindungsgemäßen Vorrichtung am Reaktionsrohr ist in Figur 4 dargestellt. An das Reaktionsrohr ist eine Halterung angeschweißt, die ein Gewinde sowie einen Vorsprung (53) aufweist, der eine umlaufende Dichtkante bildet.

Wird nun die in Figur 1 beschriebene Vorrichtung wie bereits weiter oben dargestellt in die Halterung eingeschraubt, so bildet sich eine zuverlässige Dichtung aus.

Der Rohrreaktor umfasst einen Ofen sowie ein Reaktionsrohr.

Im Allgemeinen ist ein solcher mit einem Primärenergieträger, wie mit Öl oder Gas, befeuerter Ofen in eine sogenannte Strahlungszone (16) und eine Konvektionszone (17) aufgeteilt.

In der Strahlungszone (16) wird die für die Pyrolyse erforderliche Wärme vor allem durch Strahlung der brennerbeheizten Ofenwände auf das Reaktionsrohr übertragen.

In der Konvektionszone (17) wird der Energieinhalt der heißen, aus der Strahlungszone austretenden Rauchgase durch konvektive Wärmeübertragung genutzt. So kann das Edukt der radikalischen Gasphasenreaktion vorgewärmt, verdampft oder überhitzt werden. Ebenso ist auch die Erzeugung von Wasserdampf und/oder die Vorwärmung von Verbrennungsluft möglich.

Bei einer typischen Anordnung, wie sie z.B. in EP-A-264,065 dargestellt wird, wird flüssiges Edukt der radikalischen Gasphasenreaktion zunächst in der Konvektionszone des Reaktors vorgewärmt und danach in einem speziellen Verdampfer außerhalb des Reaktors verdampft. Das dampfförmige Edukt der radikalischen Gasphasenreaktion wird dann wiederum der Konvektionszone zugeführt und dort überhitzt, wobei bereits die Pyrolysereaktion einsetzen kann.

Nach erfolgter Überhitzung tritt das Edukt der radikalischen Gasphasenreaktion in die Strahlungszone ein, wo die thermische Spaltung zum Produkt(gemisch) stattfindet.

Infolge der in der Strahlungszone und der im Eintritt der Konvektionszone herrschenden hohen Temperaturen ist es vorteilhaft, die in Figur 1 skizzierte Vorrichtung nicht direkt innerhalb dieser Zonen anzuordnen, da sonst z.B. eine definierte Temperatureinstellung des zur Förderung der Spaltreaktion eingeleiteten erhitzten und Radikale enthaltenden Gases oder Gasgemischs nicht oder nur erschwert möglich ist.

Daher wird eine Anordnung bevorzugt, wie sie in Figur 4 schematisch dargestellt ist.

Hierbei ist der Reaktor um mindestens zwei zusätzliche, nicht beheizte Kompartments (18) erweitert, die thermisch isoliert sein können. Aus der eigentlichen Strahlungs- bzw. Konvektionszone (16, 17) werden dann Schlaufen des Reaktionsrohrs durch diese Kompartments (18) geführt. In diesen Schlaufen, vorzugsweise an den Bögen der Schlaufen und mündend in die geraden Längen dieser Schlaufen, wird dann die Vorrichtung zur Einkopplung elektromagnetischer Strahlung gemäß Figur 1 (19) montiert, also in das Reaktionsrohr eingebaut, so dass an diesen Stellen der Gasstrom des Reaktionsgemischs mit elektromagnetischer Strahlung behandelt werden kann.

Die aus der Strahlungs- bzw. Konvektionszone (16, 17) in die unbeheizten Kompartments (18) geführten Schlaufen des Reaktionsrohrs sind vorzugsweise mit einer thermischen Isolation versehen. In diesem Falls müssen die Außenwände des Kompartments nicht notwendigerweise eine thermische Isolierfunktion besitzen.

## Patentansprüche

1. Vorrichtung zum Einkoppeln von elektromagnetischer Strahlung in einen Reaktor zur Durchführung radikalischer Gasphasenreaktionen, umfassend die Elemente:
a) ein vom Reaktionsraum des Reaktors abgetrenntes Kompartment, das
b) mit dem Reaktionsraum des Reaktors über mindestens eine Öffnung in Verbindung steht,
c) mindestens eine Zuleitung zum Einleiten eines Spülgases in das Kompartment, das ein Inertgas und/oder ein Spaltpromotoren enthaltendes Gas und/oder ein gasförmiges Reduktionsmittel ist, so dass sich ein Gaspolster ausbildet, welches die optischen Bauteile chemisch weitgehend vom Reaktionsraum abkoppelt, sowie
d) mindestens eine Quelle von elektromagnetischer Strahlung, die so angeordnet ist, dass
e) die elektromagnetische Strahlung das Kompartment und den daran sich anschließenden Reaktionsraum des Reaktors durchstrahlt.

2. Vorrichtung nach Anspruch 1, **dadurch gekennzeichnet, dass** diese ein optisches Fenster und/oder eine andere Lichtzuleitung in das Kompartment aufweist.

3. Vorrichtung nach Anspruch 2, **dadurch gekennzeichnet, dass** das optische Fenster und/oder der lichtdurchlässige Abschluss der anderen Lichtzuleitung mit einer optisch halbdurchlässigen Schicht beschichtet ist, die aus einem Metall besteht, das sich als Hydrierkatalysator eignet.

4. Vorrichtung nach Anspruch 1, **dadurch gekennzeichnet, dass** das Kompartment mit dem Reaktionsraum über eine Düse in Verbindung steht.

5. Vorrichtung nach Anspruch 1, **dadurch gekennzeichnet, dass** diese zwei konische Hülsen (54, 57) umfasst, die so angebracht sind, dass sich zwischen den Hülsen (54) und (57) ein Zwischenraum (67) ausbildet, der mit mindestens einer Gaszuleitung versehen ist, dass sich ein vom Reaktionsraum (68) sowie vom Außenraum (69) abgetrenntes Kompartment bildet und dass die dem Reaktor entfernter angeordnete Hülse (57) ein optisch durchlässiges Fenster (58) und/oder eine andere Lichtzuleitung enthält.

6. Vorrichtung nach Anspruch 1, **dadurch gekennzeichnet, dass** Bestrahlungsvorrichtungen vorgesehen sind, die eine Bestrahlung des gesamten Kompartments sowie des daran sich anschließenden Reaktionsraumes ermöglichen.

7. Vorrichtung nach Anspruch 5, **dadurch gekennzeichnet, dass** der Zwischenraum (67) einen weiteren Gaseinlass (72) aufweist, der bis nahe der Oberfläche des optisches Fensters und/oder der anderen Lichtzuleitung in das Kompartment geführt wird und das Spülen des optischen Fensters und/oder der anderen Lichtzuleitung und dessen Umgebung mit Inertgas oder mit Inertgas und Wasserstoff ermöglicht.

8. Vorrichtung nach Anspruch 5, **dadurch gekennzeichnet, dass** der Zwischenraum (67) einen weiteren Gaseinlass (72) aufweist, der in Richtung der Öffnung (71) geführt wird und der Zuführung einer gasförmigen Promotorsubstanz dient.

9. Reaktor zur Durchführung radikalischer Gasphasenreaktionen umfassend mindestens folgende Elemente:
i) in den Reaktor mündende Zuleitung für den Eduktgasstrom,
ii) mindestens eine ein Kompartment umfassende und in den Reaktor mündende Vorrichtung nach Anspruch 1 zur Einkopplung von elektromagnetischer Strahlung in den Reaktor
iii) in das Kompartment der Vorrichtung mündende Zuleitung für ein Spülgas, das ein Inertgas und/oder ein Spaltpromotoren enthaltendes Gas und/oder ein gasförmiges Reduktionsmittel ist, so dass sich ein Gaspolster ausbildet, welches die optischen Bauteile chemisch weitgehend vom Reaktionsraum abkoppelt,
iv) mit der Zuleitung verbundene Quelle für ein Spülgas,
v) gegebenenfalls Heizvorrichtung für das Aufheizen des Spülgases in der Zuleitung,
vi) Heizvorrichtung für das Aufheizen und/oder die Aufrechterhaltung der Temperatur des Gasstromes im Reaktor, und
vii) aus dem Reaktor führende Ableitung für den Produktgasstrom der radikalischen Gasphasenreaktion.

10. Reaktor nach Anspruch 9, **dadurch gekennzeichnet, dass** dieser ein Reaktionsrohr aufweist, an das eine ein Gewinde (52) sowie einen Vorsprung aufweisende Halterung (53) angeschweißt ist, in welche die Vorrichtung nach Anspruch 1 eingeschraubt ist.

11. Reaktor nach Anspruch 10, **dadurch gekennzeichnet, dass** dieser einen Ofen sowie ein schlaufenförmig im Ofen verlaufendes Reaktionsrohr umfasst, wobei der Ofen eine Strahlungszone (16), eine Konvektionszone (17) sowie mindestens ein nicht beheiztes Kompartment (18) aufweist, in die Schlaufen des Reaktionsrohrs aus der bzw. in die Strahlungs- bzw. Konvektionszone (16, 17) geführt werden, wobei sich die mindestens eine Vorrichtung nach Anspruch 1 in mindestens einem Kompartment (18) befindet und in das Reaktionsrohr eingebaut ist, so dass an diesen Stellen elektromagnetische Strahlung in den Gasstrom des Reaktionsgemisches eingekoppelt werden kann.

12. Reaktor nach Anspruch 9, **dadurch gekennzeichnet, das** diesem ein adiabatischer Nachreaktor nachgeschaltet ist.

13. Reaktor zur Durchführung radikalischer Gasphasenreaktionen, **dadurch gekennzeichnet, dass** dieser mit einem adiabatischen Nachreaktor enthaltend die Elemente ii), iii) und iv) nach Anspruch 9 verschaltet ist.

## Claims

1. Device for injecting electromagnetic radiation into a reactor for carrying out free-radical gas-phase reactions, which comprises the elements:
a) a compartment which is separated from the reaction space of the reactor and is
b) connected to the reaction space of the reactor via at least one opening,
c) at least one feed line for introducing a flushing gas which is an inert gas and/or a gas containing dissociation promoters and/or a gaseous reducing agent into the compartment so as to form a gas cushion which substantially decouples the optical components chemically from the reaction space, and also
d) at least one source of electromagnetic radiation which is arranged so that
e) the electromagnetic radiation passes through the compartment and the reaction space of the reactor adjoining the compartment.

2. Device according to Claim 1, **characterized in that** it has an optical window and/or another light transmission line into the compartment.

3. Device according to Claim 2, **characterized in that** the optical window and/or the transparent end of the other light transmission line is coated with an optically semitransparent layer which comprises a metal which is suitable as hydrogenation catalyst.

4. Device according to Claim 1, **characterized in that** the compartment is connected to the reaction space via a nozzle.

5. Device according to Claim 1, **characterized in that** it comprises two conical barrels (54, 57) arranged so that an intermediate space (67) which is provided with at least one gas feed line is formed between the barrels (54) and (57) so that a compartment separated from the reaction space (68) and from the outside (69) is formed and **in that** the barrel (57) arranged farthest away from the reactor contains an optically transparent window (58) and/or another light transmission line.

6. Device according to Claim 1, **characterized in that** irradiation devices which make irradiation of the entire compartment and of the adjoining reaction space possible are provided.

7. Device according to Claim 5, **characterized in that** the intermediate space (67) has a further gas inlet (72) which is continued into the compartment close to the surface of the optical window and/or the other light transmission line and makes it possible for the optical window and/or the other light transmission line and its environment to be flushed with inert gas or with inert gas and hydrogen.

8. Device according to Claim 5, **characterized in that** the intermediate space (67) has a further gas inlet (72) which is continued in the direction of the opening (71) and serves for the introduction of a gaseous promoter substance.

9. Reactor for carrying out free-radical gas-phase reactions, which comprises at least the following elements:
i) a feed line for the feed gas stream opening into the reactor,
ii) at least one device according to claim 1 comprising a compartment and opening into the reactor for injecting electromagnetic radiation into the reactor,
iii) a feed line for a flushing gas which is an inert gas and/or a gas containing dissociation promoters and/or a gaseous reducing agent, said feed line opening into the compartment of the device so as to form a gas cushion which substantially decouples the optical components chemically from the reaction space,
iv) a source of a flushing gas connected to the feed line,
v) if appropriate, a heating device for heating the flushing gas in the feed line,
vi) a heating device for heating and/or maintaining the temperature of the gas stream in the reactor, and
vii) a discharge line for the product gas stream of the free-radical gas-phase reaction leading from the reactor.

10. Reactor according to Claim 9, **characterized in that** it has a reaction tube onto which a holder (53) which has a thread (52) and a projection and into which the device according to claim 1 is screwed is welded.

11. Reactor according to Claim 10, **characterized in that** it comprises a furnace and a reaction tube running in a meandering fashion through the furnace, with the furnace having a radiation zone (16), a convection zone (17) and at least one unheated compartment (18) into which loops of the reaction tube are passed from the radiation or convection zone (16, 17) or from which the loops of the reaction tube are passed into the radiation or convection zone (16, 17), and the at least one device according to claim 1 being located in at least one compartment (18) and being installed in the reaction tube so that electromagnetic radiation can be injected into the gas stream of the reaction mixture at these points.

12. Reactor according to Claim 9, **characterized in that** an adiabatic after-reactor is installed downstream thereof.

13. Reactor for carrying out free-radical gas-phase reactions, **characterized in that** it is connected to an adiabatic after-reactor comprising the elements ii), iii) and iv) according to Claim 9.

## Revendications

1. Dispositif pour injecter du rayonnement électromagnétique dans un réacteur pour réaliser des réactions radicalaires en phase gazeuse, comprenant les éléments :
a) un compartiment séparé de la chambre de réaction du réacteur, qui
b) est en communication avec la chambre de réaction du réacteur par au moins un orifice,
c) au moins une amenée pour introduire un gaz de rinçage dans le compartiment, qui est un gaz inerte et/ou un gaz contenant des promoteurs de fission et/ou un agent de réduction gazeux, afin qu'un coussin de gaz se forme, qui découple largement les composants optiques chimiquement de la chambre de réaction, ainsi qu'au moins
d) une source de rayonnement électromagnétique qui est disposée de sorte que
e) le rayonnement électromagnétique traverse le compartiment et la chambre de réaction du réacteur raccordée à celui-ci.

2. Dispositif selon la revendication 1, **caractérisé en ce qu'**il présente une fenêtre optique et/ou une autre amenée de lumière dans le compartiment.

3. Dispositif selon la revendication 2, **caractérisé en ce que** la fenêtre optique et/ou l'extrémité perméable à la lumière de l'autre amenée de lumière est revêtue d'une couche optiquement semi-perméable qui est réalisée en un métal dit catalyseur d'hydrogénation.

4. Dispositif selon la revendication 1, **caractérisé en ce que** le compartiment est en communication avec la chambre de réaction par une buse.

5. Dispositif selon la revendication 1, **caractérisé en ce qu'**il comprend deux douilles coniques (54, 57) qui sont disposées de sorte à ce qu'un espace intermédiaire (67) se forme entre les douilles (54) et (57), qui est muni d'au moins une amenée de gaz, qu'un compartiment séparé de la chambre de réaction (68) ainsi que de l'espace extérieur (69) se forme, et la douille (57) disposée plus loin du réacteur comprend une fenêtre (58) optiquement perméable et/ou une autre amenée de lumière.

6. Dispositif selon la revendication 1. **caractérisé en ce que** des dispositifs d'irradiation sont prévus qui permettent une irradiation du compartiment complet ainsi que de la chambre de réaction raccordée à celui-ci.

7. Dispositif selon la revendication 5. **caractérisé en ce que** l'espace intermédiaire (67) présente une autre entrée de gaz (72) qui s'étend jusque près de la surface de la fenêtre optique et/ou de l'autre amenée de lumière dans le compartiment et permet le rinçage de la fenêtre optique et/ou de l'autre amenée de lumière et de son environnement par du gaz inerte ou du gaz inerte et de l'hydrogène.

8. Dispositif selon la revendication 5, **caractérisé en ce que** l'espace intermédiaire (67) présente une autre entrée de gaz (72) qui s'étend en direction de l'orifice (71) et sert à amener une substance promotrice gazeuse.

9. Réacteur pour la réalisation de réactions radicalaires en phase gazeuse, comprenant au moins les éléments suivants :
i) une amenée débouchant dans le réacteur pour le courant de gaz d'éduction,
ii) au moins un dispositif comprenant un compartiment et débouchant dans le réacteur selon la revendication 1 pour injecter du rayonnement électromagnétique dans le réacteur,
iii) une amenée débouchant dans le compartiment du dispositif pour un gaz de rinçage qui est un gaz inerte et/ou un gaz contenant des promoteurs de fission et/ou un agent de réduction gazeux, de sorte qu'un coussin de gaz se forme qui découple largement les composants optiques chimiquement de la chambre de réaction,
iv) une source reliée à l'amenée pour un gaz de rinçage,
v) le cas échéant un dispositif de chauffage pour chauffer le gaz de rinçage dans l'amenée,
vi) un dispositif de chauffage pour chauffer et/ou maintenir la température du courant de gaz dans le réacteur, et
vii) une évacuation sortant du réacteur pour le courant de gaz produit de la réaction radicalaire en phase gazeuse.

10. Réacteur selon la revendication 9, **caractérisé en ce qu'**il présente un tube de réaction sur lequel est soudé un support (53) présentant un filetage (52) ainsi qu'une saillie, dans lequel est vissé le dispositif selon la revendication 1.

11. Réacteur selon la revendication 10, **caractérisé en ce qu'**il comprend un four ainsi qu'un tube de réaction s'étendant en forme de boucle dans le four, le four présentant une zone de rayonnement (16), une zone de convection (17) ainsi qu'au moins un compartiment (18) non chauffé, dans lequel sont guidées les boucles du tube de réaction hors ou dans la zone de rayonnement ou de convection (16. 17), au moins un dispositif selon la revendication 1 étant situé dans au moins un compartiment (18) et installé dans le tube de réaction, de sorte à permettre d'injecter à ces endroits du rayonnement électromagnétique dans le courant de gaz du mélange de réaction.

12. Réacteur selon la revendication 9, **caractérisé en ce qu'**un réacteur subséquent adiabatique est placé en aval de celui-ci.

13. Réacteur pour la réalisation de réactions radicalaires en phase gazeuse, **caractérisé en ce que** celui-ci est relié à un réacteur subséquent adiabatique comprenant les éléments ii), iii) et iv) selon la revendication 9.
